Europäisches Patentamt

European Patent Office

Office européen des brevets

(19)

(11) EP 0 839 523 A2

(12) EUROPÄISCHE PATENTANMELDUNG

(43) Veröffentlichungstag:
06.05.1998 Patentblatt 1998/19

(51) Int. Cl.⁶: A61K 7/50, A61K 7/48

(21) Anmeldenummer: 97116146.8

(22) Anmeldetag: 17.09.1997

(84) Benannte Vertragsstaaten:
AT BE CH DE DK ES FI FR GB GR IE IT LI LU MC NL PT SE

(30) Priorität: 26.09.1996 DE 19639598

(71) Anmelder:
Henkel Kommanditgesellschaft auf Aktien
40589 Düsseldorf-Holthausen (DE)

(72) Erfinder:
• Gassenmeier, Thomas, Dr.
  40229 Düsseldorf (DE)
• Millhoff, Jürgen, Dipl.-Ing.
  40211 Düsseldorf (DE)
• Busch, Peter, Dr. Dipl.-Chem.
  40699 Erkrath (DE)
• Hensen, Hermann, Dr. Dipl.-Chem.
  42781 Haan (DE)

(54) Körperreinigungsmittel

(57) Reinigungsmittel auf Basis von anionischen Tensiden enthalten zur Verbesserung der Hautverträglichkeit einen Zusatz von wenigstens einem Lipid und wenigstens einem wasserlöslichen Polypeptid oder Polypeptidderivat. Bevorzugt liegt das Gewichtsverhältnis von anioschem Tensid (A) zu Lipid (B) und wasserlöslichem Polypeptid (C) im Bereich von A : B : C = 1 : 0,01 - 0,4 : 0,01 - 0,4. Als Lipid eignet sich bevorzugt ein Fettsäure-polyol-partialester und als wasserlösliches Polypeptid ein Abbauprodukt von Kollagen, Soja- oder Getreideprotein.

EP 0 839 523 A2

**Beschreibung**

Die Erfindung betrifft Reinigungsmittel mit einem Gehalt an anionischen Tensiden, die zur Verbesserung der Hautverträglichkeit und zur Verringerung der Hautaustrocknung Zusätze von Lipiden und wasserlöslichen Polypeptiden enthalten.

Die in Körperreinigungsmitteln gebräuchlichen synthetischen Tenside, insbesondere die in Shampoos, Dusch- und Schaumbad-Formulierungen und Flüssigseifen gebräuchlichen, stark schäumenden anionischen Sulfattenside haben den Nachteil, daß sie zu einer starken Entfettung der Haut und zu einer mehr oder weniger nachhaltigen Quellung der oberen Hautschichten beitragen. In der Folge führt dies zu einem verstärkten Wasserverlust, so daß die Haut einen trockenen und rauhen Eindruck macht. Es hat daher nicht an Versuchen gefehlt, diese unerwünschten Nebenwirkungen der stark schäumenden Tenside durch geeignete Zusätze von Ölkomponenten oder Rückfettungsmitteln oder von sogenannten Hautfeuchthaltungsmitteln zu verringern.

So ist es z.B. bekannt, daß Fettstoffe, die in den Körperreinigungsmitteln emulgiert oder solubilisiert sind, eine gewisse Rückfettung bewirken. Auch von wasserlöslichen Proteinen ist bekannt, daß sie als Zusätze in Tensidformulierungen eine Verbesserung der Haut- und Schleimhautverträglichkeit bewirken.

Aus US-A-4,306,997 war z.B. bekannt, daß Fettsäuremonoglyceride in Kombination mit anionischen Tensiden zu einer Verbesserung des Hautgefühls beitragen, ohne die Schaumwirkung zu beeinträchtigen.

Daß wasserlösliche Proteine eine hautschützende Wirkung in wäßrigen Tensidzubereitungen haben, war z.B. schon aus der deutschen Offenlegungsschrift DE-A-16 17 062 bekannt. Auch synthetische Lipoproteine sind zur Verbesserung der haarkosmetischen Effekte gemäß DE-A-23 30 176 schon in Shampoos auf Basis anionischer Tenside eingesetzt worden.

Es bestand aber nach wie vor ein Bedürfnis, die Hautverträglichkeit von Körperreinigungsmitteln und anderen wäßrigen Reinigungsmitteln, z.B. von manuellen Geschirrspülmitteln, die häufig mit der ungeschützten Haut in Kontakt kommen, durch geeignete Zusätze weiter zu verbessern. Insbesondere sollte die starke Quellung der Haut mit der Folge des anschließenden starken Wasserverlustes weiter verringert werden.

Es wurde nunmehr gefunden, daß wäßrige Reinigungsmittel mit einem Gehalt an anionischen Tensiden (A) durch den Zusatz einer Kombination spezieller Lipide (B) und wasserlöslicher Polypeptide (C) besonders spürbar in ihrer Hautverträglichkeit verbessert werden. Insbesondere wird der transepidermale Wasserverlust nach der Tensideinwirkung erheblich verringert. Der Effekt ist so stark, daß er nicht als eine additive Wirkung der an sich bekannten Zusatzmittel zu verstehen ist.

Gegenstand der Erfindung sind daher Reinigungsmittel mit einem Gehalt an anionischen Tensiden (A) und Zusätzen zur Verbesserung der Hautverträglichkeit, dadurch gekennzeichnet, daß als Zusätze wenigstens ein Lipid (B) und wenigstens ein wasserlösliches Polypeptid oder Polypeptid-Derivat (C) enthalten ist.

Als erfindungsgemäße Reinigungsmittel werden insbesondere solche Produkte verstanden, die zur Körperreinigung geeignet sind oder die bei der Anwendung mit der Haut in Kontakt kommen. Solche Produkte sind z.B. Schaum- und Duschbad-Zusammensetzungen, Shampoos, flüssige Seifen, manuelle Geschirrspülmittel und flüssige Haushaltsreiniger. Als anionische Tenside (A) sind in solchen Produkten bevorzugt schaumstarke synthetische Tenside mit einer anionischen Sulfonat- oder Sulfatgruppe enthalten. Geeignete Beispiele solcher Tenside sind z.B. Alkylbenzolsulfonate mit 12 - 18 C-Atomen, Alkylsulfate mit 12 - 18 C-Atomen, Sulfobernsteinsäuremono- und -diester-Salze, Acylisethionate, Acyltauride, die Fettsäuremonoglycerid-sulfate von $C_{12}$-$C_{18}$-Fettsäuren oder die Alkylpolyglycolethersulfate mit 12 - 16 C-Atomen in der Alkylgruppe und 1 - 10 Glycolethergruppen (die sogenannten Ethersulfate).

Andere geeignete Aniontenside, die allein oder im Gemisch mit den vorgenannten Sulfonat- oder Sulfattensiden enthalten sein können, sind z.B. die Fettsäureseifen von $C_{12}$-$C_{18}$-Fettsäuren, die Acylsarkoside mit $C_{12}$-$C_{18}$-Acylgruppen, die Alkylpolyglycolethercarboxylate mit 12 - 18 C-Atomen in der Alkylgruppe und 1 - 6 Glycolethergruppen (die sogenannten Ethercarboxylate) und die Alkylphosphate und Alkylpolyglycoletherphosphate.

Die anionischen Tenside liegen als wasserlösliche Salze, z.B. als Alkali-, Magnesium-, Ammonium- oder Alkanolammonium-Salze vor. Die anionischen Tenside sind in den erfindungsgemäßen Reinigungsmitteln bevorzugt in einer Menge von 1 - 30 Gew.-% enthalten. Es sind aber auch Reiniger-Konzentrate mit Gehalten bis zu 80 Gew.-% der anionischen Tenside mit flüssiger oder pastöser Konsistenz herstellbar.

Als Lipide (B) eignen sich alle in Wasser unlöslichen, aber in wäßrigen Tensidlösungen solubilisierbaren Fettstoffe. Bevorzugt sind dabei solche Fettstoffe, die eine oder zwei lineare Alkylgruppen mit 12 - 22 C-Atomen und eine oder mehrere polare Gruppen, z.B. Carboxyl-, Hydroxyl- oder Ethergruppen aufweisen. Beispiele für solche geeigneten Lipide sind z.B. Fettalkohole, 1.2-Alkandiole, Fettsäuren und Fettsäureamide mit jeweils 12 - 18 C-Atomen, Fettsäuremonoalkanolamide, Glycerinmonoalkylether und Di-n-Alkylether. Besonders bevorzugt geeignete Lipide sind die Fettsäure-polyol-partialester aus $C_{12}$-$C_{18}$-Fettsäuren und $C_2$-$C_6$-Polyolen mit 2 - 6 Hydroxylgruppen. Beispiele für solche Lipide sind z.B. Ethylenglycol-monolaurat, Glycerin-monooleat oder Sorbitan-sesquistearat.

Als Polyol-Partialester eignen sich auch technische Produkte, die in der Regel Gemische aus Mono- und Diestern solcher Polyole darstellen. Monoester mit hohem Anteil von mehr als 90 Gew.-% des Monoesters sind besonders bevorzugt.

Als wasserlösliche Polypeptide (C) eignen sich z.B. wasserlösliche Proteine wie z.B. Albumin oder Proteinabbauprodukte, die durch alkalische, saure oder enzymatische Hydrolyse aus wasserunlöslichen tierischen oder pflanzlichen Proteinen erhältlich sind. Diese Produkte sollten, um das Merkmal der Wasserlöslichkeit zu erfüllen, in Wasser in einem pH-Bereich von pH = 5 - 8 bei 20°C zu wenigstens 1 Gew.-% löslich sein. Die Proteinhydrolysate sollten dabei jedoch nicht völlig bis zur Stufe der Aminosäuren abgebaut sein, vielmehr sollten die wasserlöslichen Polypeptide noch mittlere Molekulargewichte von mehr als 500 D aufweisen.

Als wasserlösliche Polypeptide eignen sich auch Derivate von Protein-Abbauprodukten, z.B. mit $C_{12}$-$C_{18}$-Fettsäuren acylierte Proteinhydrolysate, die in Form ihrer Salze wasserlöslich sind. Bevorzugt eignen sich als wasserlösliche Polypeptide (C) die Abbauprodukte von Kollagen, Soja- oder Getreideprotein. Geeignete Polypeptide dieser Art sind im Handel erhältlich, z.B. unter den Warenzeichen Gluadin®W40 (Weizenprotein-Hydrolysat), Lexein X 350 (Kollagen-Hydrolysat), Crosilk®1000 (Seidenprotein-Hydrolysat), Phytokine (Sojaprotein-Hydrolysat), Gelita-Sol-P (Gelatine-Hydrolysat), Nutrilan-L (Kollagen-Hydrolysat).

Das Lipid (B) und das Polypeptid (C) sollten in den erfindungsgemäßen Reinigungsmitteln nur als Zusätze, also in deutlich geringeren Mengen als das Aniontensid (A) enthalten sein. Bevorzugt liegt das Gewichtsverhältnis von anionischem Tensid (A) zu Liquid (B) und wasserlöslichem Polypeptid (C) im Bereich von (A) : (B) : (C) = 1 : 0,01 - 0,4 : 0,01 - 0,4.

In einer bevorzugten Ausführung betrifft die Erfindung Körperreinigungsmittel in Form einer wäßrigen Zubereitung mit einem Gehalt von

2 - 20 Gew.-%     eines Sulfattensids der Formel
R - O $(C_2H_4O)_n$ - $SO_3^{(-)}M^{(+)}$, in der R eine lineare Alkylgruppe mit 10 bis 16 C-Atomen, n = 0 oder eine Zahl von 1 bis 10 und $M^{(+)}$ ein Alkalimetall, $Mg^{(++)}/2$ oder Ammoniumion ist

0,5 - 5 Gew.-%     eines Monoglycerids einer Fettsäure mit 12 - 22 C-Atomen und

0,5 - 5 Gew.-%     eines wasserlöslichen Proteinhydrolysats

vorliegt.

Die erfindungsgemäßen Reinigungsmittel können als wasserfreie oder wasserarme Konzentrate vorliegen und eine feste, pastöse oder gelartige Konsistenz aufweisen. Bevorzugt liegen die erfindungsgemäßen Reinigungsmittel jedoch als fließfähige, wäßrige Lösungen vor. In diesen Zubereitungen ist das Lipid durch das Aniontensid klar solubilisiert, gegebenenfalls wird die Solubilisierung durch Mitverwendung weiterer Tenside oder Emulgatoren erreicht.

Solche Reinigungsmittel sind beim Kontakt mit der Haut besonders verträglich und verringern die durch das Tensid bedingte Quellung der Epidermis sowie den anschießenden transepidermalen Wasserverlust.

Ein weiterer Gegenstand der Erfindung ist ein Verfahren zur Verbesserung der Hautverträglichkeit anionischer Tenside durch Zusätze, wobei man zu 1 Gewichtsteil der anionischen Tenside (A) 0,1 bis 0,5 Gewichtsteile eines Lipids (B) und 0,1 bis 0,5 Gewichtsteile eines wasserlöslichen Polypeptids oder Polypeptidderivates zusetzt.

Die erfindungsgemäßen Reinigungsmittel können neben den genannten, kennzeichnenden Komponenten noch weitere Hilfsmittel enthalten, die in solchen Produkten üblich sind. Als solche sind z.B. Duftstoffe, dermatologische Wirkstoffe, haarkosmetische Zusätze (z.B. in Shampoos), Farbstoffe, Trübungsmittel, Konservierungsmittel, pH-Wert-Stellmittel, Verdickungsmittel, z.B. wasserlösiche Polymere, Elektrolyte und andere übliche Hilfsmittel enthalten. Die Menge solcher weiteren Zusätze sollte aber nicht mehr als 10 Gew.-% des Reinigungsmittels ausmachen.

Die Herstellung der erfindungsgemäßen Reinigungsmittel erfolgt durch einfaches Vermischen der Komponenten, dabei ist es von Vorteil, bei der Herstellung wäßriger Zubereitungen die Komponenten in erwärmtem Wasser, etwa bei 40 - 80°C, zu lösen.

Die folgenden Beispiele sollen den Gegenstand der Erfindung näher erläutern:

**Beispiele**

**1. Vergleichende gravimetrische Bestimmung des transepidermalen Wasserverlustes.**

**1.1 Versuchsmethodik**

Frische oder frisch aufgetaute Schweinehautlappen werden mechanisch (mit einem Messer) vom Fettgewebe getrennt und in eine Petrischale gedrückt, so daß ein Teil der Schweinehaut den Boden bedeckt und der Rest an den Seiten übersteht. Dann wird auf die Schweinehaut die zu prüfende Testlösung aufgegeben und bei 40°C über 45 Minuten einwirken gelassen. Danach wird der Schweinehautlappen mit 0,9 %iger NaCl-Lösung abgespült. Schließlich werden aus dem behandelten Areal (am Boden der Petrischale) und dem unbehandelten (überstehenden) Areal kreisrunde Probestücke ($\varnothing$ 25 mm) ausgestanzt.

Die Probestücke werden in einem speziellen Probenbehälter über einer 0,9 %igen NaCl-Lösung so fixiert, daß die Hautunterseite mit dieser Kochsalzlösung und die Hautoberseite mit der umgebenden Atmosphäre in Kontakt ist. Der Probenbehälter wird in eine klimatisierte Wiegeeinrichtung eingehängt und dort bei 36°C in wasserfreier $N_2$-Atmosphäre zuerst 3 Stunden konditioniert (zur Gleichgewichtseinstellung), dann gewogen und nach weiteren 3 Stunden erneut gewogen.

Die Gewichtsdifferenz zwischen beiden Messungen gibt die in dieser Zeit durch die Hautprobe diffundierte Wassermenge an, die dann in mg/cm$^2$/h umgerechnet wird.

## 1.2 Versuchsauswertung

Der transepidermale Wasserverlust (TEWL) ist stark von der Art der Hautprobe abhängig. Daher wird stets der Quotient

$$\frac{TEWL\ (Probe)\ \cdot\ 100}{TEWL\ (unbehandelt)} = \%\ TEWL\ (Probe)\ errechnet.$$

Dieser Wert wird wiederum für eine Standard--Tensidlösung (12 Gew.-% Fettalkohol ($C_{12}$-$C_{14}$)-polyglycolether-(2 EO)-sulfat, Na-Salz) und für eine Probelösung an benachbarten Arealen der gleichen Hautprobe ermittelt und der Quotient aus

$$\frac{\%\ TEWL\ (Probe)}{\%\ TEWL\ (Tensid)} = rel.\ TEWL$$

gebildet. Dieser Wert stellt ein Maß für den Einfluß der Zusätze auf den TEWL einer Tensidlösung dar.

## 1.3 Versuchsergebnisse

| Rezepturen | V1 | V2 | V3 | 1 |
|---|---|---|---|---|
| Texapon®NSO (1) | 43,0 | 43,0 | 43,0 | 43,0 |
| Monomuls 90-0-18 (2) | - | 1,8 | - | 1,8 |
| Gluadin W 40 (3) | - | - | 5,0 | 5,0 |
| Lamepon LPO (4) | - | - | - | - |
| Wasser | 57,0 | 55,2 | 52,0 | 50,2 |
| rel. TEWL | 1 | 12,2 | 2,6 | 0,08 |

## 2. Anwendungsbeispiele

|  | 1 | 2 | 3 | 4 | 5 | 6 |
|---|---|---|---|---|---|---|
| Texapon® N 25 | 30 | 20 | 15 | 10 | 20 | 18 |
| Dehyton®K | 5 | - | 10 | 10 | - | 6 |
| Plantacare®-1200-UP | - | 5 | - | 5 | - | - |
| Monomuls®90-0-18 | 1,5 | - | - | - | 2,0 | - |
| Propylenglycolmonolaurylether | - | 1 | - | - | - | - |
| Cetiol® | - | - | 2,0 | - | - | 1 |
| 2-Octyl-dodecanol | - | - | - | 1 | - | 1 |
| Gluadin®W40 | 10,0 | - | 5,0 | 5,0 | - | - |
| Nutrilan®-Pulver | - | - | - | - | 2,0 | - |
| Hydromond® | - | 2,0 | - | - | - | 1,5 |
| Parfümöl | 0,7 | 0,7 | 0,7 | 0,7 | 0,7 | 0,7 |
| Konservierungsmittel | 0,3 | 0,3 | 0,3 | 0,3 | 0,3 | 0,3 |
| Wasser | 64,0 | 71,0 | 67,0 | 68,0 | 75,0 | 71,5 |

Es wurden die folgende Handelsprodukte verwendet:

| | |
|---|---|
| Texapon®N 25 | : Fettalkohol ($C_{12}/C_{14}$)-polyglycolether-(2 EO)-sulfat, Na-Salz (28 %ig in $H_2O$) (Henkel KGaA) |
| Dehyton®K | : Kokosalkylamidopropyl-dimethyl-acetobetain (30 %ig in $H_2O$) (Henkel KGaA) |
| Plantacare 1200 - UP | : Alkyl-($C_{10}$-$C_{16}$)-oligo-(1.4)-glucosid (50 %ig in $H_2O$) (Henkel KGaA) |
| Monomuls-90-0-18 | : Glycerinmonooleat (Henkel/Grünau) |
| Cetiol®OE | : Di-n-octylether (Henkel) |
| Gluadin® W-40 | : Weizenproteinhydrolysat (Henkel/Grünau) (40 %ig in $H_2O$) |
| Hydromond® | : Mandelproteinhydrolysat (CRODA) |

## Patentansprüche

1. Reinigungsmittel mit einem Gehalt aninischen Tensiden (A) und Zusätzen zur Verbesserung der Hautverträglichkeit, dadurch gekennzeichnet, daß als Zusätze wenigstens ein Lipid (B) und wenigstens ein wasserlösliches Polypeptid oder Polypeptidderivat (C) enthalten ist.

2. Reinigungsmittel nach Anspruch 1, dadurch gekennzeichnet, daß als anionisches Tensid (A) ein synthetisches Tensid mit einer anionischen Sulfonat- oder Sulfatgruppe enthalten ist.

3. Reinigungsmittel nach Anspruch 1 oder 2, dadurch gekennzeichnet, daß das Gewichtsverhältnis von anionischem Tensid (A) zu Lipid (B) und wasserlöslichem Polypeptid (C) im Bereich von A : B : C = 1 : 0,01 - 0,4 : 0,01 - 0,4 liegt.

4. Reinigungsmittel nach einem der Ansprüche 1 - 3, dadurch gekennzeichnet, daß als Lipid ein Fettsäure-polyol-partialester aus $C_{12}$-$C_{18}$-Fettsäuren und $C_2$ - $C_6$ - Polyolen mit 2 - 6 Hydroxylgruppen enthalten ist.

5. Körperreinigungsmittel nach einem der Ansprüche 1 - 4, dadurch gekennzeichnet, daß das wasserlösliche Polypeptid (C) ein Abbauprodukt von Kollagen, Soja- oder Getreideprotein ist.

6. Körperreinigungsmittel nach einem der Ansprüche 1 - 5, dadurch gekennzeichnet, daß es als wäßrige Zubereitung mit einem Gehalt von

2 - 20 Gew.-%     eines Sulfattensids der Formel
R - O $(C_2H_4O)_n$ - $SO_3^{(-)}$ $M^{(+)}$, in der R eine lineare Alkylgruppe mit 10 bis 16 C-Atomen, n = 0 oder eine Zahl von 1 bis 10 und $M^{(+)}$ ein Alkalimetall, $Mg^{(++)}$/2 oder Ammoniumion ist

0,5 - 5 Gew.-%     eines Monoglycerids einer Fettsäure mit 12 - 22 C-Atomen und

0,5 - 5 Gew.-%     eines wasserlöslichen Proteinhydrolysats

vorliegt.

7. Verfahren zur Verbesserung der Hautverträglichkeit anionischer Tenside durch Zusätze, dadurch gekennzeichnet, daß man zu 1 Gewichtsteil der anionischen Tenside 0,1 bis 0,5 Gewichtsteile eines Lipids und 0,1 bis 0,5 Gewichtsteile eines wasserlöslichen Polypeptids zusetzt.